# EUROPEAN PATENT SPECIFICATION

(11) **EP 1 366 729 B1**
(45) Date of publication and mention of the grant of the patent: **27.07.2005**
(21) Application number: 03014514.8
(22) Date of filing: 21.05.1996
(51) Int. Cl.: A61F 2/00, A61F 6/20, A61B 17/12, A61B 17/42, A61F 6/22

(54) **Contraceptive transcervical fallopian tube occlusion devices having mechanical fallopian tube attachment**
Transcervikale Verhütungsvorrichtung zum Eileiterverschluss mit mechanischer Befestigung am Eileiter
Dispositifs contraceptifs d'occlusion de la trompe de fallope par le canal cervical, à fixation mécanique à la trompe

(30) Priority: 07.06.1995 US 475252
(43) Date of publication of application: 03.12.2003
(62) Divisional of application: 96920409.8
(73) Proprietor: CONCEPTUS, INC., San Carlos, CA 94070 (US)
(72) Inventor: Nikolchev, Julian, Portola Valley, CA 94028 (US); Ton, Dai, Milpitas, CA 95035 (US)
(74) Representative: Merrifield, Sarah Elizabeth

(56) References cited:
- EP-A- 0 105 669
- WO-A-94/06503
- US-A- 3 805 767
- US-A- 3 858 586

## Description

### BACKGROUND OF THE INVENTION

### 1. Field of the Invention

The present invention relates generally to contraception, and more particularly to intrafallopian contraceptive devices.

Worldwide demand exists for safe, effective methods of both contraception and permanent sterilization. Although a variety of contraception and sterilization methods are available, all of the existing methods have limitations and disadvantages. Thus, the need for additional safe, low cost, reliable methods of contraception and permanent sterilization, both in developed and less developed countries, is widely recognized.

Many presently available contraception methods require significant user involvement, and user non-compliance results in quite high rates of failure. While the theoretical effectiveness of existing contraceptives, including barrier methods and hormonal therapies, is well established, overcoming user noncompliance to improve overall efficacy has proven difficult.

One form of contraception which is less susceptible to user noncompliance is the intrauterine device (IUD). IUDs have been found to have higher rates of reliability, and are effective for a longer period of time, than most other commercially available contraceptives. Unfortunately, IUDs are also associated with serious infectious complications. For this reason, the use of IUDs within the United States has decreased dramatically. Additionally, IUDs are subject to unplanned expulsion, and must be removed due to excessive pain or bleeding in a percentage of cases, further reducing the acceptance of the IUD as a contraceptive method. Interestingly, the efficacy of copper IUDs appears to be higher than that of non-metallic IUDs. The reason for this has not been fully explained.

Commercially available options for permanent sterilization include fallopian tube ligation and vasectomy. These methods are surgical, are difficult to reverse, and are not available to many people in the world. It is common knowledge that fertilization occurs in the fallopian tubes where the sperm and ovum meet. Tubal ligation avoids this by complete occlusion of the fallopian tubes.

It has previously been proposed to reversibly occlude the fallopian tubes, for example, by *in vitro* formation of an elastomeric plug, or otherwise anchoring a device on either side of the narrowest region of fallopian tube, called the "isthmus." Such fallopian tube occlusion methods appear promising; however, an unacceptably high percentage of the non-surgical devices proposed to date have become dislodged during previous studies. Even where non-surgical intrafallopian devices have remained in place, they have been found to be only moderately effective at preventing conception.

For these reasons, it would be desirable to provide effective, reliable intrafallopian devices for contraception and sterilization. It would be particularly desirable to provide highly effective intrafallopian devices which did not require surgery for placement. It would be especially desirable if such devices and methods allowed easy placement of the device, but were less susceptible to being dislodged than previously proposed non-surgical intrafallopian devices.

### 2. Description of the Related Art

The experimental use of a stainless steel intrafallopian device is described in *Transcatheter Tubal Sterilization in Rabbits,* Penny L. Ross, RT 29 "Investigative Radiology", pp. 570-573 (1994). The experimental use of an electrolytically pure copper wire as a surgical contraceptive intrafallopian device in rats was described in "Antifertility Effect of an Intrafallopian Tubal Copper Device", D.N. Gupta, 14 *Indian Journal of Experimental Biology,* pp. 316-319 (May 1976).

U.K. Patent Application Pub. No. 2,211,095 describes a uterine screw plug for blocking the fallopian tube. European Patent Application Pub. No. 0,010,812 describes a device for placement in the oviducts having enlargements at either end for anchoring the device. The same device appears to be described in Netherlands Patent No. 7,810,696.

The use of tubal occlusion devices is described in "Hysteroscopic Oviduct Blocking With Formed-in-Place Silicone Rubber Plugs", Robert A. Erb, Ph.D., et al., *The Journal of Reproductive Medicine,* pp. 65-68 (August 1979). A formed-in-place elastomeric tubal occlusion device is described in U.S. Patent No. 3,805,767, issued to Erb. U.S. Patent No. 5,065,751, issued to Wolf, describes a method and apparatus for reversibly occluding a biological tube. U.S. Patent No. 4,612,924, issued to Cimber, describes an intrauterine contraceptive device which seals the mouths of the fallopian tubes.

German Patent No. 28 03 685, issued to Brundin, describes a device for plugging a body duct with a device which swells when in contact with a body fluid.

Document WO 94/06503 discloses a detachable embolic coil assembly, in which coils for delivery to a site within the vasculature are linked together on a control wire by means of clasps or hooks.

Document EP 0105669 discloses an expansible plug which can be inserted into a fallopian tube as a method of contraception. The plug expands into a sealing contact with the wall of the body duct.

### SUMMARY OF THE INVENTION

The present invention provides intrafallopian devices to prevent conception. The intrafallopian devices of the present invention are transcevically delivered and mechanically anchored within the fallopian tube to provide long term contraception, or alternatively permanent sterilization, without the need for surgical procedures or the risks of increased bleeding, pain, and infection associated with intrauterine devices (IUDs).

The intrafallopian devices of the present invention generally comprise a structure having a lumen-traversing region with a helical outer surface. The helical surface is mechanically anchored by a resilient portion of the structure which is biased to form an enlarged secondary shape, preferably forming distal and proximal anchoring loops. The anchoring loops help prevent the helical outer surface from rotating out of position, and also directly deter axial motion within the fallopian tube.

The use of copper in the intrafallopian device of the present invention improves its efficacy as a contraceptive method. Devices formed from plastically deformable materials, however, are less readily restrained in the fallopian tube. Apparently, the large variation in the actual shape and dimensions of fallopian tubes does not provide reliable anchoring for a pre-formed deformable intrafallopian device. The intrafallopian device of the present invention therefore comprises a resilient structure, usually a metallic coil, which includes a copper alloy or plating, ideally comprising an alloy including at least 75% copper. The coil material typically includes beryllium, zinc, stainless steel, platinum, a shape memory alloy, such as Nitinol™, or the like. Preferably, the coil is composed of an alloy of beryllium and copper. Although the present device will generally result in occlusion, it need not completely occlude the fallopian tube to prevent the meeting of the sperm and ovum. Instead, the presence of the copper on the resilient structure is sufficient to provide effective contraception.

Conveniently, the present invention further comprises non-surgical placement of such intrafallopian devices by transcervical introduction. The resilient structure is restrainable in a straight configuration, e.g., by use of a corewire, greatly facilitating and reducing the risks of introduction. Thus, the cost and dangers associated with existing surgical contraceptive and sterilization procedures are avoided.

The present invention provides an intrafallopian contraceptive device comprising: a resilient structure restrained in a straight configuration with a corewire extending into the resilient structure, the resilient structure including a lumen-traversing region have a helical outer surface; the resilient structure adapted for introduction transcervically into a target region of a fallopian tube; and the corewire withdrawable from the resilient structure so as to mechanically anchor the resilient structure within the fallopian tube, at least a portion of the resilient structure assuming an enlarged secondary shape; and the corewire is capable of transmitting torque to the resilient structure so as to anchor the resilient structure.

Preferably, the lumen-traversing region comprises a resilient structure, generally having a ribbon wound over the outer surface to form the helical shape. Anchoring is enhanced by a sharp outer edge on the ribbon. As described above, at least one of the proximal anchor, the distal anchor, and the lumen-traversing region preferably comprises copper. The proximal and distal anchors generally comprise a resilient structure biased to form an enlarged secondary shape, thereby allowing the device to be restrained in a straight configuration to facilitate transcervical introduction.

In another aspect, a contraceptive intrafallopian device according to the present invention comprises a primary coil having a proximal loop, a distal loop, and an intermediate straight section between the loops. A helical ribbon is wound over at least a portion of the intermediate section, forming a helical surface to mechanically anchor the device within the fallopian tube.

The ribbon of the present intrafallopian device generally protrudes sufficiently to firmly engage the tubal wall. Preferably, the ribbon has a width in the range between .005 and .1 inch (0.01 and 0.3 cm), a thickness in the range between .001 and .2 inch (0.003 and 0.5 cm), and a pitch in the range between .01 and .2 inch (0.03 and 0.5 cm). The overall device geometry preferably facilitates introduction and retention, but is not large or rigid enough to interfere with internal tissue movements. Usually, the device has a length in the range between 1.5 cm and 7.5 cm when in a relaxed state, while the distal loop and the proximal loop have outer diameters of at least 3 mm. Preferably, the primary coil has an outer diameter in the range between .2 mm and 5 mm.

In another aspect, a system for delivering intrafallopian contraceptive devices according to the present invention comprises a primary coil having a proximal loop, a distal loop, and an intermediate straight section between the loops. Additionally, a lumen extends from a proximal end of the proximal loop to near a distal end of the distal loop. A helical ribbon is wound over at least a portion of the intermediate section, forming a helical surface to mechanically anchor the device within the fallopian tube. A corewire is removably disposed within the lumen of the primary coil. The corewire restrains the primary coil in a straight configuration, facilitating trancervical introduction. Optionally, the corewire is threadably received by the primary coil. Alternatively, a release catheter is slidably disposed over the corewire proximally of the primary coil to restrain the primary coil while the corewire is withdrawn proximally from the fallopian tube.

The helical ribbon is anchored in the fallopian tube by the distal and proximal loops. The ribbon is set in the tubal wall while the device is restrained in a straight configuration over a corewire by torquing on the corewire. Withdrawing of the corewire then releases the anchors. The distal anchor is generally inserted into the ampulla, distal of the isthmus, while the proximal anchor is located in the ostium. These anchors prevent rotation of the device, and also help avoid axial movement.

In yet another aspect, an intrafallopian contraceptive method according to the principles of the present invention comprises restraining a resilient contraceptive structure in a straight configuration over a corewire, where the resilient structure includes a lumen-traversing region having a helical outer surface. The resilient structure is transcervically introduced into a target region of a fallopian tube, typically in the region of the ostium, and the corewire is withdrawn from the resilient structure. The resilient structure is mechanically anchored within the fallopian tube, a portion of the resilient structure assuming an enlarged secondary shape which is larger in cross-section than the fallopian tube. Optionally, an electric current is applied through the resilient structure to the fallopian tube, thereby effecting permanent sterilization.

### BRIEF DESCRIPTION OF THE DRAWINGS

Fig. 1 illustrates a first embodiment of a contraceptive intrafallopian device according to the present invention.
Fig. 2 illustrates a primary coil used in the contraceptive intrafallopian device of Fig. 1.
Fig. 3 illustrates a secondary coil which has been imposed on a primary coil as used in the contraceptive intrafallopian device of Fig. 1.
Fig. 4 illustrates a corewire for use with the contraceptive intrafallopian device of Fig. 1.
Fig. 5 is a cross-sectional view of a contraceptive delivery system having the contraceptive intrafallopian device of Fig. 1.
Fig. 6 illustrates an alternative embodiment of the present contraceptive intrafallopian device.
Fig. 7 illustrates a primary coil used in the contraceptive intrafallopian device of Fig. 6.
Fig. 8 schematically illustrates a contraceptive delivery system including the contraceptive intrafallopian device of Fig. 6.
Figs. 9 and 10 illustrates a method of delivery of a contraceptive intrafallopian device according to the present invention.

### DETAILED DESCRIPTION OF THE SPECIFIC EMBODIMENT

The present invention encompasses a contraceptive intrafallopian device which can alternatively be used as both a permanent and a reversible means of contraception. The present contraceptive methods and devices minimize the danger of non-use which has limited the efficacy of prior art contraceptive techniques. Moreover, the location of the present devices within the fallopian tubes provides a reduced risk of the infectious complications, increased bleeding, and pelvic pain associated with intrauterine devices (IUDs). The location and the novel shape of the present intrafallopian device provides significant advantages over IUDs, which have been found to be susceptible to unplanned expulsion and removal due to excessive pain and bleeding. The present invention takes advantage of the increase in effectiveness associated with copper IUDs, providing a resilient structure including copper which may be transcervically positioned without the need for surgery.

Although the present contraceptive method is included within a group of contraceptive techniques generally referred to as fallopian tube occlusion methods, the present invention does not necessarily rely solely on blocking the fallopian tube to prevent fertilization. Instead, contraception is apparently provided by disrupting of ovum transport, the process of fertilization, and/or cleavage of the ovum. While the effect that copper has on these processes is not fully understood, it does appear that copper intrafallopian devices offer potentially significant increases in effectiveness over intrafallopian devices formed of other materials. Optionally, the present invention further encompasses devices which promote the growth of tissue within the tube to induce tubal occlusion, further inhibiting conception.

Conveniently, the present resilient structures are adapted to be releasably affixed over a corewire, the corewire restraining the resilient structure in a straight configuration. As the resilient structure has an outer diameter when in the straight configuration which is less than the inner diameter of the fallopian tube, the catheter containing the present intrafallopian device is easily transcervically introduced.

The present invention is anchored within the isthmus ' of the fallopian tube, overcoming the unintended expulsion of the device and the resulting failure of the contraceptive method. Such intrafallopian device expulsion has been the single greatest factor limiting the efficacy of easily positioned intrafallopian contraceptive techniques. The present intrafallopian devices are generally elongate resilient structures pre-formed into secondary shapes. These secondary shapes will preferably form anchors proximally and distally of the narrowest portion of the fallopian tube, called the isthmus. The secondary shape must have a larger outer diameter than the inner diameter of the isthmus.

The present device is generally readily removed by snaring the resilient structure near the proximal end and pulling proximally on the resilient structure, thereby straightening the resilient structure and allowing it to be withdrawn without injuring the fallopian tube. Alternatively, an electrical current is applied to the device after it is positioned within the fallopian tube, providing permanent sterilization.

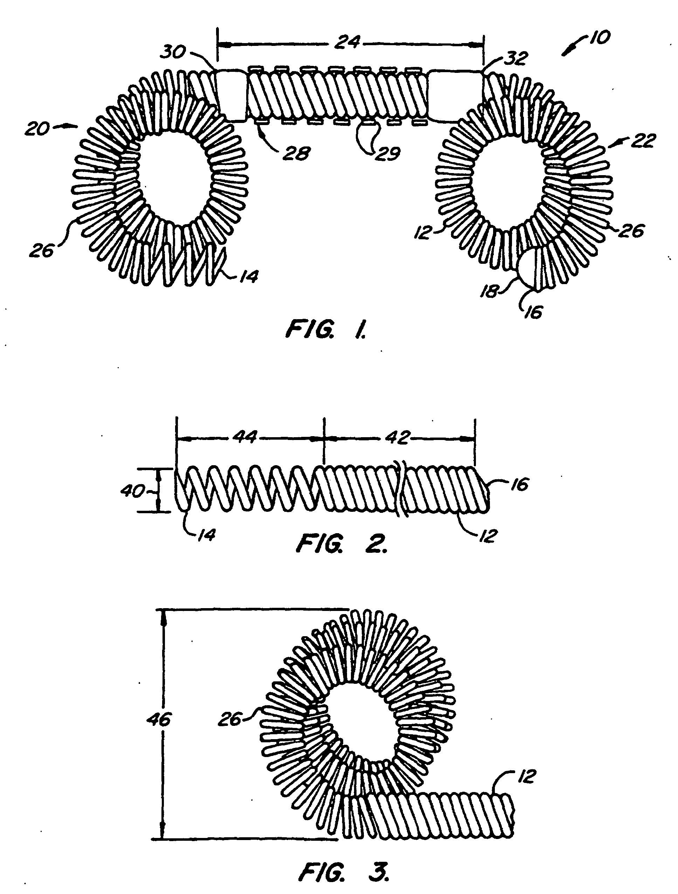

Referring now to Fig. 1, a first embodiment of the present contraceptive intrafallopian device 10 is formed from a resilient primary coil 12. Primary coil 12 has a proximal end 14 and a distal end 16, the latter having an atraumatic endcap 18. Primary coil 12 further includes three portions: a proximal anchor portion 20, a distal anchor portion 22, and a lumen-traversing region 24. Proximal and distal anchors 20,22 are biased to form anchoring loops 26, as described hereinbelow.

Lumen-traversing region 24 comprises a substantially straight portion of primary coil 12. A ribbon 28 is wound over the outer surface of primary coil 12 to provide a helical shape. Ribbon 28 includes sharp outer edges 29, which firmly anchor lumen-traversing region 24 in the fallopian tube wall when torque is applied to intrafallopian device 10. The ribbon is preferably formed of a high strength biocompatible metal, ideally being stainless steel. The ribbon is attached to primary coil 12 at a proximal joint 30 and a distal joint 32, which may be formed of solder, heat-shrink tubing, or the like.

Referring now to Fig. 2, primary coil 12 is most easily formed in a straight configuration as a cylindrical coil or spring, preferably having an outer diameter in the range from .005 inch to .05 inch (0.01 to 0.1 cm), and having a length in the range from 20 mm to 150 mm. Ideally, primary coil 12 has an outer diameter in the range from .01 inch to .05 inch (0.03 to 0.1 cm) and a length in the range from 30 mm to 125 mm.

Preferably, primary coil 12 is formed from a beryllium copper alloy wire. Beryllium copper provides the resilience necessary to avoid expulsion of the device, and also provides the increased effectiveness of a copper contraceptive intrafallopian device. Such a beryllium copper wire will typically have a diameter from .002 inch to .01 inch. To provide the increased efficacy of a copper intrafallopian device, primary coil 12 preferably comprises an alloy including 75% copper. Alternatively, primary coil 12 is formed from a resilient metal, such as stainless steel, platinum, a shape memory alloy, or the like. If such materials are used, primary coil 12 is preferably plated with copper or a copper alloy or otherwise has copper attached.

Primary coil 12 includes a body winding 42 and a thread winding 44. Body winding 42 is formed with the minimum possible pitch to increase the stiffness of primary coil 12. Thread winding 44 will typically comprise from 0.1 cm to 2 cm adjacent to proximal end 14, and will have a pitch roughly twice that of body winding 42.

Referring now to Fig. 3, the proximal and distal anchors are formed by imposing a bent secondary shape on selected portions of primary coil 12. The secondary shape preferably comprises loops 26 formed by bending primary coil 12, and heat treating the primary coil while it is bent. A wide variety of secondary shapes may be used, including sinusoidal curves, alternating loops, or loops separated by straight sections so as to form a "flower coil".

In all cases, the bent secondary shape should have an outer cross-section 46 which is larger than the fallopian tube to provide effective anchoring.

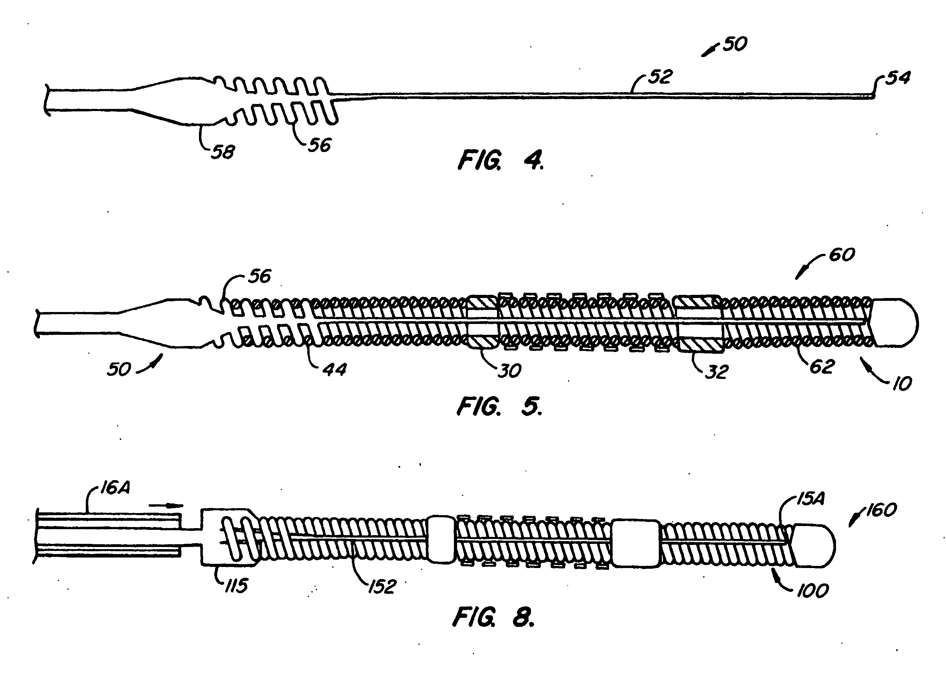

Referring now to Fig. 4, a corewire 50 for use with intrafallopian device 10 (Fig. 1) comprises a resilient wire 52 which tapers towards a distal end 54. Wire 52 is sufficiently stiff to restrain intrafallopian device 10 in a straight configuration, typically comprising stainless steel, platinum, or the like. A short section of coil forms corewire threads 56 attached at threadjoint 58. Threads 56 match the windings and pitch of threadwindings 44 of primary coil 12.

Referring now to Fig. 5, an intrafallopian contraceptive system 60 comprises corewire 50 inserted within a lumen 62 through intrafallopian device 10. Intrafallopian device 10 is releasably attached by engaging thread windings 44 with threads 56. Thus, intrafallopian device 10 is disengaged by torquing a proximal end of corewire 50 once intrafallopian device 10 is in position.

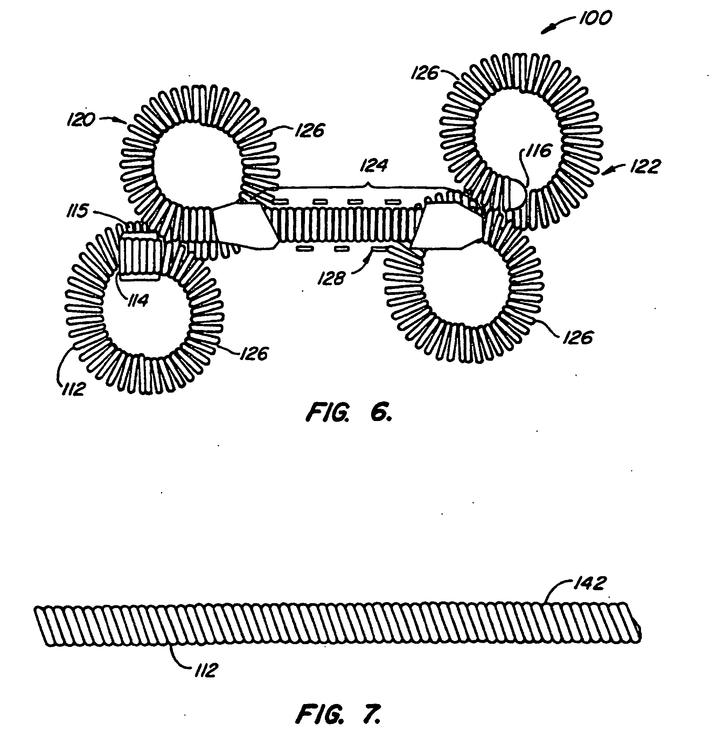

Referring now to Fig. 6, an alternative embodiment of the present intrafallopian device is again formed from a resilient primary coil 112 having a proximal end 114 and a distal end 116. The former includes a friction fitting 115. Primary coil 112 again includes three portions: a proximal anchor portion 120, a distal anchor portion 122, and a lumen-traversing region 124. Proximal and distal anchors 120, 122 are here biased to form opposed anchoring loops 26, thereby increasing the relaxed overall cross-section of the proximal and distal anchors. A ribbon 128 is wound over the outer surface of primary coil 112 to provide a helical shape, as described above.

Referring now to Fig. 7, primary coil 112 comprises a uniform body winding 142. The secondary shape is imposed on the straight cylindrical coil as opposed loops 126, or alternatively as multiple loops of a flower coil.

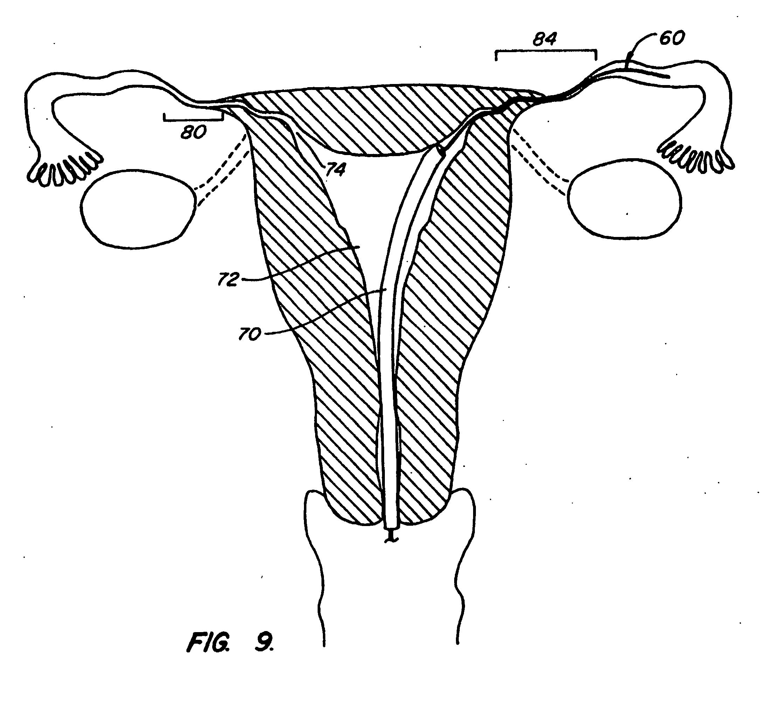

Referring now to Fig. 8, an intrafallopian contraceptive system using alternative intrafallopian device 100 includes a corewire 152 which tapers towards a distal end 154. Friction fitting 115 fittingly engages corewire 152, which restrains primary coil 112 in a straight configuration. A release catheter 164 is slidably disposed over corewire 152 proximally of alternative intrafallopian device 100, allowing the device to be released by withdrawing corewire 152 relative to the release catheter.

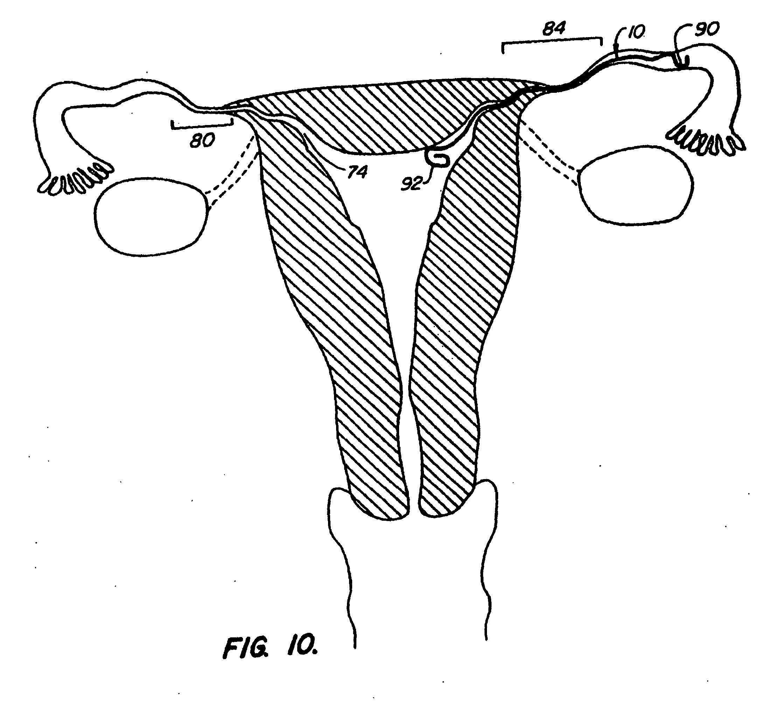

Use of the present contraceptive intrafallopian device will be described with reference to Figs. 9 and 10. A uterine introducer canula 70 is inserted transcervically through a uterus 72 to the region of an ostium 74. Alternatively, a hysteroscope may be used in place of canula 70.

Intrafallopian contraceptive system 60 is advanced distally of introducer cannula 70 and manuevered through the fallopian tube, preferably until intrafallopian device 10 extends distally of the isthmus. Optionally, intrafallopian contraceptive system 60 is self-guided, with corewire 52 bent near distal end 54 to assist intraluminal manuevering. Alternatively, a guide wire and catheter are advanced into the fallopian tube first, and the guide wire is replaced with intrafallopian contraceptive system 60. In either case, the intrafallopian device is axially positioned with lumen-traversing region 24 within a target region 84 adjacent to isthmus 80. Preferably, at least one loop of distal anchor 22 is distal of target region 84, and at least one loop of proximal anchor 20 is proximal of target region 84 to form the distal and proximal anchor bends.

Once intrafallopian device 10 is properly positioned, corewire 50 is torqued to set ribbon 28 in the tubal wall. The corewire may then be unthreaded from intrafallopian device 10 by rotating the corewire in the opposite direction, disengaging threads 56 from thread windings 44. The corewire is then free to slide proximally, releasing the primary coil. As the distal end of the primary coil is released, a distal anchor bend 90 is formed. Similarly, a proximal loop forms a proximal anchor bend 92. The anchor bends help to axially restrain the device within the fallopian tube, and also prevent rotation around the helical shape of lumen-traversing region 24. As seen in Fig. 10, the loops need not assume their relaxed form to provide effective distal or proximal anchors.

The present invention further encompasses permanent sterilization by passing a current through the corewire to the intrafallopian device prior to withdrawing the corewire. Fallopian tube tissue in contact with the intrafallopian device is dessechated, and thus attached to the present intrafallopian device. This action also causes permanent tubal damage, leading to the formation of scar tissue which encapsulates the intrafallopian device and causes permanent occlusion of the tubal lumen. Clearly, the corewire/primary coil interface must be conductive to allow the present non-surgical method of permanent sterilization.

In conclusion, the present invention provides a contraceptive intrafallopian device which may be positioned without surgery. While the above is a complete description of the preferred embodiments of the invention, various alternatives, modifications, and equivalents may be used. For example, a wide variety of secondary shapes, including open loops, continuous bends, sinusoidal curves, or the like, may be imposed on the primary coil. Therefore, the above description should not be taken as limiting the scope of the invention, which is defined instead solely by the appended claims.

## Claims

1. An intrafallopian contraceptive device comprising:
a resilient structure restrained in a straight configuration with a corewire (50) extending into the resilient structure, the resilient structure including a lumen-traversing region (24) having a helical outer surface;
the resilient structure adapted for introduction transcervically into a target region of a fallopian tube;
the corewire (50) withdrawable from the resilient structure so as to mechanically anchor the resilient structure within the fallopian tube, at least a portion of the resilient structure assuming an enlarged secondary shape which is larger in cross-section than the fallopian tube; and
the corewire (50) is capable of transmitting torque to the resilient structure so as to anchor the resilient structure in the tubular wall.

2. An intrafallopian contraceptive device as claimed in claim 1, wherein withdrawal of the corewire (50) forms a distal anchor from a portion of the resilient structure which is distal of the lumen-traversing region, and forms a proximal anchor from a portion of the resilient structure which is proximal of the lumen-traversing region (24), the distal portion and the proximal portion assuming a secondary shape.

3. An intrafallopian contraceptive device as claimed in claim 1, wherein the corewire (50) is releasably threaded to the resilient structure.

4. An intrafallopian contraceptive device as claimed in claim 1, wherein the resilient structure is axially restrained with a release catheter while the corewire (50) is releasably threaded to the resilient structure, the release catheter being slidably disposed over the corewire (50).

5. An intrafallopian contraceptive device as claimed in claim 1, further comprising a device which promotes the growth of tissue within the fallopian tube to induce tubal occlusion of the fallopian tube.

## Patentansprüche

1. In den Eileiter einzusetzende kontrazeptive Vorrichtung, umfassend:
eine elastische Struktur, die mit einem sich in der elastischen Struktur erstreckenden Kerndraht (50) in einer geraden Konfiguration zurückgehalten wird, wobei die elastische Struktur einen Lumen-querenden Bereich (24) mit einer gewendelten Außenoberfläche aufweist;
wobei die elastische Struktur zum transzervikalen Einführen in einen Zielbereich eines Eileiters ausgelegt ist;
wobei der Kerndraht (50) aus der elastischen Struktur herausziehbar ist, um die elastische Struktur in dem Eileiter mechanisch zu verankern, wobei zumindest ein Abschnitt der elastischen Struktur eine vergrößerte Sekundärform einnimmt, die im Querschnitt größer ist als der Eileiter; und
wobei der Kerndraht (50) in der Lage ist, auf die elastische Struktur ein Drehmoment auszuüben, um die elastische Struktur in der Gefäßwand zu verankern.

2. In den Eileiter einzusetzende kontrazeptive Vorrichtung gemäß Anspruch 1, worin das Herausziehen der Kerndrahts (50) aus einem Abschnitt der elastischen Struktur, der distal des Lumen-querenden Bereichs ist, einen distalen Anker bildet, und aus einem Abschnitt der elastischen Struktur, die proximal des Lumen-querenden Bereichs (24) ist, einen proximalen Anker bildet, wobei der distale Abschnitt und der proximale Abschnitt eine Sekundärform einnehmen.

3. In den Eileiter einzusetzende kontrazeptive Vorrichtung gemäß Anspruch 1, worin der Kerndraht (50) lösbar an die elastische Struktur geschraubt ist.

4. In den Eileiter einzusetzende kontrazeptive Vorrichtung gemäß Anspruch 1, worin die elastische Struktur mit einem Ausgabekatheter axial zurückgehalten wird, während der Kerndraht (50) lösbar an die elastische Struktur geschraubt ist, wobei der Ausgabekatheter verschiebbar über dem Kerndraht (50) angeordnet ist.

5. In den Eileiter einzusetzende kontrazeptive Vorrichtung gemäß Anspruch 1, die ferner eine Vorrichtung umfasst, die das Gewebewachstum in dem Eileiter fördert, um einen Tubusverschluss des Eileiters zu induzieren.

## Revendications

1. Dispositif contraceptif intrafallopien, comprenant :
une structure élastique maintenue en configuration droite avec un fil métallique d'âme (50) s'étendant dans la structure élastique, la structure élastique comprenant une région traversée par une lumière (24) ayant une surface extérieure hélicoïdale ;
la structure élastique étant adaptée à l'introduction transcervicale dans une région cible d'une trompe de Fallope ;
le fil métallique d'âme (50) pouvant être retiré de la structure élastique de manière à ancrer mécaniquement la structure élastique dans la trompe de Fallope, au moins une partie de la structure élastique acquérant une forme secondaire agrandie qui est plus grande en section transversale que la trompe de Fallope ; et
le fil métallique d'âme (50) étant capable de transmettre un moment de torsion à la structure élastique de manière à ancrer la structure résiliente dans la paroi tubulaire.

2. Dispositif contraceptif intrafallopien suivant la revendication 1, dans lequel le retrait du fil métallique d'âme (50) forme une ancre distale à partir d'une partie de la structure élastique qui est en position distale de la région parcourue par une lumière, et forme une ancre proximale à partir d'une partie de la structure élastique qui est en position proximale de la région traversée par une lumière (24), la partie distale et la partie proximale acquérant une forme secondaire.

3. Dispositif contraceptif intrafallopien suivant la revendication 1, dans lequel le fil métallique d'âme (50) est raccordé par filetage de manière amovible à la structure élastique.

4. Dispositif contraceptif intrafallopien suivant la revendication 1, dans lequel la structure élastique est maintenue axialement avec un cathéter de séparation tandis que le fil métallique d'âme (50) est raccordé par filetage de manière amovible à la structure élastique, le cathéter de séparation étant disposé de manière à pouvoir glisser sur le fil métallique d'âme (50).

5. Dispositif contraceptif intrafallopien suivant la revendication 1, comprenant en outre un dispositif qui favorise la croissance de tissu dans la trompe de Fallope pour induire une occlusion tubaire de la trompe de Fallope.
